# EUROPÄISCHE PATENTSCHRIFT

(11) **EP 4 073 266 B1**
(45) Veröffentlichungstag und Bekanntmachung des Hinweises auf die Patenterteilung: **27.05.2026**
(21) Anmeldenummer: 20833739.4
(22) Anmeldetag: 10.12.2020
(51) Int. Cl.: C12Q 1/6818, C12Q 1/6825

(54) **VERFAHREN ZUR DURCHFÜHRUNG EINER AMPLIFIKATIONSREAKTION IN EINER MIKROFLUIDISCHEN VORRICHTUNG**
METHOD FOR CARRYING OUT AN AMPLIFICATION REACTION IN A MICROFLUIDIC APPARATUS
PROCÉDÉ POUR METTRE EN OEUVRE UNE RÉACTION D'AMPLIFICATION DANS UN DISPOSITIF MICROFLUIDIQUE

(30) Priorität: 13.12.2019 DE 102019219531
(43) Veröffentlichungstag der Anmeldung: 19.10.2022
(73) Patentinhaber: Robert Bosch GmbH, 70442 Stuttgart (DE)
(72) Erfinder: LAERMER, Franz, 71263 Weil Der Stadt (DE); SCHULZ, Martin, 73650 Winterbach (DE)
(86) Internationale Anmeldenummer: PCT/EP2020/085602
(87) Internationale Veröffentlichungsnummer: WO 2021/116322

(56) Entgegenhaltungen:
- WO-A1-2004/033726
- WO-A2-2008/072209
- GB-A- 2 365 866
- US-A1- 2019 195 806

## Beschreibung

Die vorliegende Erfindung betrifft ein Verfahren zur Durchführung einer Amplifikationsreaktion in einer mikrofluidischen Vorrichtung, wobei die Reaktion unter Verwendung von mit Fluorophor und Quencher markierten Ausgangssubstanzen und einem chemischen Energieträger zur Anregung der Fluorophore mittels Energieübertrag durchgeführt wird. Weiterhin betrifft die Erfindung einen Kit und eine mikrofluidische Vorrichtung zur Durchführung einer solchen Amplifikationsreaktion.

### Stand der Technik

Bei der Polymerase-Kettenreaktion (PCR) handelt es sich um eine in den 80er Jahren entwickelte schnelle und sehr sensitive Methode in der DNA-Analytik. Mit Hilfe von spezifischen Primern wird hierbei eine definierte enzymatische *in vitro-*Replikation der DNA durchgeführt, bei der die Zielsequenz in mehreren Zyklen vervielfältigt (amplifiziert) wird. Nach der ursprünglich verwendeten radioaktiven Markierung der Reaktionsprodukte hat sich hierbei in der Zwischenzeit die Verwendung von Fluoreszenz zum Nachweis der Reaktionsprodukte durchgesetzt. Hierfür kann die Reaktion unter Verwendung von mit Fluorophor und einem Quencher markierten Ausgangssubstanzen durchgeführt werden. Zur Erzeugung der Fluoreszenz werden die Fluorophore bzw. fluoreszierenden Farbstoffe mit einer entsprechenden Anregungswellenlänge optisch angeregt. Wenn sich in der räumlichen Nähe des Fluorophors ein Quencher befindet, wird die Fluoreszenz strahlungslos deaktiviert. Wenn Fluorophor und Quencher im Rahmen der erfolgenden Amplifikationsreaktion räumlich voneinander getrennt werden, ist die Fluoreszenz messbar.

Es können für die Reaktion beispielsweise mit einem Fluorophor markierte Primerketten oder sogenannte TaqMan-Sonden verwendet werden, welche ein Fluorophor und einen Quencher in räumlicher Nähe enthalten. Während der Polymerase-Ketten-Reaktion werden diese Ketten oder Sonden eingebaut und/oder hydrolysiert, so dass die räumliche Nähe zwischen Fluorophor und Quencher aufgelöst wird und eine Fluoreszenz aus den Reaktionsprodukten nach optischer Anregung nachweisbar ist. Das Anregungslicht und die Fluoreszenzemission haben in der Regel unterschiedliche Wellenlängen (sogenannte "Stokes-Verschiebung" zwischen Absorptions- und Emissionsmaxima). Mit Hilfe von optischen Filtern in einer Detektionseinrichtung können diese Wellenlängen voneinander separiert werden, so dass ein Detektor oder eine Detektionskamera in Idealfall nur das Licht mit der Wellenlänge der Fluoreszenzemission detektiert. Dies wird beispielsweise in der WO 2004/033726 A1 beschrieben.

Die GB 2 365 866 A beschreibt die Verwendung photolumineszenter Nukleinsäureketten als Fluorophore.

In der WO 2008/072209 A2 wird eine Mikrovorrichtung für die Durchführung einer PCR-Analyse beschrieben. Ein photolumineszentes Polymer ist am Boden einer Analysekammer angeordnet. Dieses kann mittels Bestrahlung zur Lumineszenz angeregt werden. Die Lumineszenzwellenlänge entspricht der Anregungswellenlänge eines als Sonde verwendeten Fluorophors.

### Offenbarung der Erfindung

### Vorteile der Erfindung

Das vorgeschlagene Verfahren dient zur Durchführung einer Amplifikationsreaktion in einer mikrofluidischen Vorrichtung, beispielsweise auf einem Lab-on-a-Chip innerhalb von sogenannten Silizium-Mikroarrayzellen. Derartige mikrofluidische Vorrichtungen werden bereits beispielsweise in der medizinischen Diagnostik verwendet. Die Amplifikationsreaktion wird unter Verwendung von mit Fluorophor und Quencher markierten Ausgangssubstanzen durchgeführt. Im Rahmen der Amplifikationsreaktion erfolgt eine Trennung von Fluorophor und Quencher, wodurch ein Nachweis von Reaktionsprodukten bzw. eine Auswertung der erfolgten Amplifikation durch die detektierbare Fluoreszenzemission möglich ist. Gemäß dem vorgeschlagenen Verfahren erfolgt der Nachweis anhand der Fluoreszenzemission durch Zugabe von wenigstens einer chemolumineszierenden Substanz und Auswertung der erfolgenden Fluoreszenzemission der Fluorophore. Anders als bei herkömmlichen Verfahren ist es bei dem vorgeschlagenen Verfahren nicht notwendig, dass das Probenvolumen optisch angeregt wird, um eine Auslesung der Fluoreszenz zu ermöglichen. Vielmehr erfolgt die Anregung der Fluorophore durch Übertragung von chemischer Energie mit Hilfe der chemolumineszierenden Substanz.

Der Chemolumineszenz liegt zugrunde, dass entsprechende chemolumineszierende Substanzen chemisch in einen angeregten Energiezustand versetzt werden können. Der erwähnte angeregte Energiezustand der chemolumineszierenden Substanzen ist in der Regel langlebig, d.h. in der Regel handelt es sich um Energiezustände, von denen ausgehend ein optischer Übergang in einen Grundzustand z.B. aufgrund von Dipolauswahlregeln verboten ist. Beispielsweise kann es sich bei dem angeregten Energiezustand um einen sogenannten Triplettzustand (T1) handeln, von dem aus ein optischer Übergang in einen Singulettgrundzustand (S0) verboten ist. Die vorliegende Erfindung nutzt die verhältnismäßig langlebige chemische Anregbarkeit von chemolumineszierenden Substanzen, da die Energie aus den langlebigen angeregten Zuständen besonders leicht auf andere Moleküle übertragen werden kann, um beispielsweise die erwähnten Fluorophore durch Energie-Übertragung zur Fluoreszenzemission anzuregen. Die Anregung der chemolumineszierenden Substanzen selbst erfolgt beispielsweise durch eine Oxidationsreaktion mit oder ohne Katalysator. Kernpunkt des vorgeschlagenen Verfahrens ist also, dass nicht eine optische Anregung der Fluoreszenz erfolgt, sondern dass mit Hilfe von chemolumineszierenden Substanzen Energie auf die Fluorophore übertragen wird, so dass die Fluoreszenzemission der Fluorophore in Abwesenheit von Quenchern in der unmittelbaren Nachbarschaft auswertbar ist. Die Anregungsenergie wird also an die gequenchten bzw. nicht-gequenchten Fluorophore nach der Amplifikationsreaktion über eine chemolumineszierende Substanz, zugeführt. Zweckmäßigerweise sollten darüber hinaus die als Energie-Überträger wirkenden Substanzen wasserlöslich sein, damit sie in Puffersystemen, wie sie für derartige Reaktionen verwendet werden, einsetzbar sind.

Durch diese neuartige Kombination eines chemische Energie tragenden Moleküls mit geeigneten Fluorophoren kann auf eine optische Anregung verzichtet werden. Eine optische Anregung zur Erzeugung der Fluoreszenz ist insbesondere bei mikrofluidischen Vorrichtungen oftmals problematisch bzw. aufwendig, da eine möglichst homogene Lichteinstrahlung einer definierten Intensität und Wellenlänge über eine möglichst große Fläche der mikrofluidischen Vorrichtung erfolgen muss. Dies erfordert aufwendige und teure Optiken, welche bei mikrofluidischen Vorrichtungen insbesondere im Hinblick auf Point-of-Care-Geräte und/oder Geräte mit hohem Durchsatz aus Kostengründen nicht gewünscht sind. Durch die Verwendung von chemolumineszierenden Substanzen gemäß dem vorgeschlagenen Verfahren zur Anregung der Fluorophore kann auf solche aufwendigen und teuren Optiken bei der Durchführung der Amplifikationsreaktionen verzichtet werden. Das vorgeschlagene Verfahren erlaubt also ein optisches Ausleseverfahren für eine Amplifikationsreaktion, beispielsweise für eine PCR-Reaktion und/oder eine isothermale Amplifikationsreaktion, in mikrofluidischen Vorrichtungen, ohne dass eine optische Lichtquelle und entsprechende optische Einrichtungen zur Anregung einer Lichtemission erforderlich wären.

Bei der chemolumineszierenden Substanz kann es sich beispielsweise um 3-Aminophthalhydrazid (5-Amino-2,3-dihydrophthalazin-1,4-dion; bekannt als Luminol) und/oder 3-Nitrophthalhydrazid handeln. Derartige chemolumineszierende Substanzen zeichnen sich dadurch aus, dass sie durch zugeführte Energie insbesondere über eine Oxidationsreaktion in einen angeregten Zustand versetzt werden und dabei Licht emittieren. Da es sich um langlebige angeregte Energiezustände handelt, ist die Chemolumineszenz der erwähnten Substanzen vergleichsweise schwach. Die der Lichtemission (Lumineszenzstrahlung) entsprechende Wellenlänge bzw. der entsprechende Energiebetrag wird im Rahmen des vorgeschlagenen Verfahrens dazu genutzt, um strahlungslos auf die Fluorophore übertragen zu werden und so die Fluoreszenz der von den Quenchern getrennten Fluorophore anzuregen. Die Energie-Übertragung erfolgt dabei im Prinzip unabhängig von der Lumineszenzstrahlung.

Die Reaktion, die zur Molekülanregung und nachfolgend zur Energieübertragung führt, basiert insbesondere auf einer Oxidationsreaktion. Insofern bezieht die chemolumineszierende Substanz ihre chemische Anregungsenergie insbesondere aus einer (katalysierten) Oxidationsreaktion. Als Oxidationsmittel kann insbesondere Wasserstoffperoxid oder Carbamidperoxid (= Wasserstoffperoxid-Harnstoff) eingesetzt werden, so dass der Nachweis im Rahmen des vorgeschlagenen Verfahrens vorzugsweise in Gegenwart von Wasserstoffperoxid durchgeführt wird. Die besonders bevorzugte chemolumineszierende Substanz Luminol wird als chemischer Energieträger durch eine Oxidation mit Wasserstoffperoxid in einen langlebigen angeregten (Triplett-) Zustand überführt. Diese gespeicherte Anregungsenergie kann dann auf andere, insbesondere längerwelliger emittierende Fluorophore übertragen werden, so dass die Fluoreszenz als Nachweis der Amplifikationsreaktion ermöglicht wird. Dieser Energieübertrag aus dem nur schwach lumineszierenden Luminol oder verwandter Moleküle auf ein stark fluoreszierendes Molekül, wie z.B. ein Rhodamin oder Yakima-Yellow, führt insgesamt zu einer Steigerung der Lichtemission, weshalb man auch von "Chemisensibilisierung" spricht.

Die Oxidationsreaktion, die zur Anregung der chemolumineszierenden Substanzen führt, wird dabei durch den Zerfall von Wasserstoffperoxid getrieben. Das Wasserstoffperoxid kann beispielsweise in Form von Carbamidperoxid vorliegen, also als Gemisch von Wasserstoffperoxid und Urea (Harnstoff). Weiterhin können zusätzlich oder alternativ für den Nachweis alkalische Bedingungen eingestellt werden, wobei beispielsweise in der Reaktionsflüssigkeit, die die chemolumineszierende Substanz enthält, wenigstens eine alkalische Substanz enthalten ist. Beispielsweise kann verdünnte Natronlauge oder Natriumhydrogencarbonatlösung vorgesehen sein, welche die Löslichkeit der chemolumineszierenden Substanzen, wie z.B. Luminol, steigert und die gleichzeitig den Zerfall von Wasserstoffperoxid beschleunigt. Alternativ können entsprechende Puffer verwendet werden.

Mit besonderem Vorteil erfolgt der Nachweis in Gegenwart von wenigstens einem Katalysator, der die dem energieübertragenden Vorgang zugrunde liegende chemische Reaktion katalysiert. Vorzugsweise katalysiert der Katalysator also eine Oxidationsreaktion. Bei dem Katalysator kann es sich vorzugsweise um Kaliumhexacyanoferrat-III und/oder Manganperoxid und/oder Hydrochinon oder verwandte Substanzen, z. B. Brenzcatechin oder Resorcin, handeln. Da manche Katalysatoren nicht mit der eigentlichen Amplifikationsreaktion interagieren, kann es in besonders bevorzugten Ausführungsformen vorgesehen sein, dass ein solcher PCR (oder Amplifikations)-kompatibler Katalysator in der mikrofluidischen Vorrichtung der Mikroarrayzellen bereits vorgelegt ist. In diesem Zusammenhang ist beispielsweise Hydrochinon besonders geeignet. In anderen Ausgestaltungen können beispielsweise enzymatische Katalysatoren, z. B. *Horseradish Peroxidase* (HRP), eingesetzt werden.

Vorzugsweise erfolgt die Zugabe der wenigstens einen chemolumineszierenden Substanz durch Überschichtung der mikrofluidischen Vorrichtung (beispielsweise des Silizium-Mikroarray-Chips) mit einer Reaktionsflüssigkeit, in der die wenigstens eine chemolumineszierende Substanz und gegebenenfalls weitere Komponenten, wie z.B. Wasserstoffperoxid und gegebenenfalls ein Katalysator, enthalten sind. Mit Überschichtung der mikrofluidischen Vorrichtung ist hierbei gemeint, dass die Reaktionsflüssigkeit insbesondere nach Abschluss der Amplifikationsreaktion in die mikrofluidische Vorrichtung eingeleitet wird, so dass eine Überschichtung oberhalb der Reaktionsräume der mikrofluidischen Vorrichtung stattfindet. Mit Reaktionsflüssigkeit ist hierbei die Flüssigkeit gemeint, in der die Anregung der chemolumineszierenden Substanzen erfolgt, also insbesondere die Anregung der chemolumineszierenden Substanzen. Vorzugsweise handelt es sich hierbei um eine Oxidationsreaktion zur Anregung der chemolumineszierenden Substanzen, welche nachfolgend ihre chemische Energie auf die Fluorophore übertragen können. Eine Überschichtung hat den besonderen Vorteil, dass die Oxidationsreaktion und die Energieübertragung auf die Fluorophore im Prinzip nur an der Grenzfläche der Flüssigkeiten stattfinden, wo sich die unterschiedlichen Komponenten durch Diffusion vermischen, so dass die Reaktion, die für die Auswertung genutzt wird, zeitlich besser ausgenutzt werden kann und die erzeugte Fluoreszenz über einen längeren Zeitraum detektiert werden kann.

Bei dieser Ausgestaltung des vorgeschlagenen Verfahrens handelt sich also um die Verwendung einer chemisch angeregten Lumineszenzreaktion zur Anregung der Fluoreszenz, die als *Readout* bzw. Nachweisreaktion für eine Amplifikationsreaktion eingesetzt wird. Diese Kombination ist im Hinblick auf mikrofluidische Vorrichtungen sehr vorteilhaft, da die für mikrofluidische Vorrichtungen oftmals nur schwierig zu realisierende optische Anregung verzichtbar ist und durch eine Zugabe der entsprechenden Reagenzien für die Lumineszenzreaktion ersetzt werden kann. Dies kann in einfacher Weise durch Überschichten mit einer entsprechenden Reaktionsflüssigkeit nach dem Abschluss der Amplifikationsreaktion im Sinne einer Endpunktreaktion durchgeführt werden. Ein solches Überschichten ist in einfacher Weise für eine Automatisierung geeignet, so dass dieses Verfahren besondere Vorteile im Hinblick auf die Verwendung von mikrofluidischen Vorrichtungen, beispielsweise in der medizinischen Diagnostik, mit hohem Durchsatz und mit wenig apparativem Aufwand geeignet ist.

Als Fluorophore eignen sich allgemein fluoreszierende Farbstoffe, die in einem Bereich mit längerer Wellenlänge als die verwendeten chemolumineszierenden Substanzen emittieren. Dies hat den Vorteil, dass die störende lumineszierende Strahlung und die fluoreszierende Strahlung, die für die eigentliche Auswertung von Interesse ist, durch optische Filter leicht voneinander getrennt werden können, so dass die störende lumineszierende Hintergrund-Strahlung für die Auswertung gewissermaßen ausgeblendet werden kann. Als Fluorophore eignen sich insbesondere Rhodamin-B und/oder andere Rhodamine und/oder Yakima-Yellow und/oder Cy-5, wobei Yakima-Yellow besonders bevorzugt ist. Das Emissionsmaximum von Yakima-Yellow liegt bei 550 nm und erstreckt sich bis über 600 nm hinaus. Das Absorptionsmaximum von Yakima-Yellow liegt bei 525 nm. Das Maximum der Lumineszenz-Emission von Luminol liegt bei 425 nm und langwelliger, so dass ein strahlungsloser Energieübertrag auf Yakima-Yellow leicht stattfinden kann. Die Wellenlänge der Fluoreszenzemission von Yakima-Yellow (550 nm oder mehr) und die Wellenlänge der "störenden" Lumineszenzemission von Luminol (425 nm oder mehr) unterscheiden sich jedoch so deutlich, dass sie durch einen optischen Filter leicht voneinander getrennt werden können, so dass die Lumineszenzemission von Luminol für die Auswertung ausgeblendet werden kann.

In besonders bevorzugten Ausgestaltungen des Verfahrens erfolgt die Auswertung durch Verwendung von wenigstens einem optischen Filter zur Abtrennung von störender lumineszierender Strahlung von der Fluoreszenzstrahlung. Zweckmäßigerweise wird der optische Filter an die jeweilig verwendeten Substanzen und deren Emissionswellenlängen, also insbesondere die Lumineszenz-Emissionswellenlänge und die Fluoreszenz-Emissionswellenlänge, angepasst, so dass im Fall von lumineszierenden Substanzen die Lumineszenz-Emissionswellenlänge herausgefiltert wird. Zur Filterung kann beispielsweise ein optisches Bandfilter mit einer Durchlasswellenlänge von z.B. 570 nm und/oder ein optisches Kantenfilter mit einer Durchlasswellenlänge ab z.B. 570 nm oder längerwellig verwendet werden, so dass im Fall der Verwendung von Luminol und Yakima-Yellow die beiden Emissionen dieses Beispiels mit besonders hoher Effizienz separiert werden können.

In einer besonders bevorzugten Ausgestaltung handelt es sich bei der Amplifikationsreaktion um eine Endpunkt-Reaktion, also insbesondere um eine Endpunkt-PCR oder um eine Endpunktreaktion einer isothermalen Amplifikationsreaktion. Bei einer Durchführung des Verfahrens "auf Endpunkt" erfolgt kein *Readout* während des Verlaufs der Amplifikationsreaktion, sondern das Ende der Amplifikationsreaktion wird abgewartet, bis dann die Nachweisreaktion durch Zugabe der entsprechenden Substanzen bzw. Überschichten mit den chemolumineszierenden Substanzen und der für die Initiierung der Lumineszenzreaktion erforderlichen Substanzen, gestartet wird. Nach Ablauf der Amplifikationsreaktion, also wenn die Vervielfältigungsreaktion in den Reaktionsräumen der mikrofluidischen Vorrichtung an ihrem Endpunkt angekommen ist, kann die mikrofluidische Vorrichtung mit der Reaktionsflüssigkeit, die die chemolumineszierenden Substanzen und gegebenenfalls weitere Substanzen enthält, wie beispielsweise einen Katalysator und Wasserstoffperoxid als Oxidationsmittel, überschichtet werden. Es ist auch möglich und vorteilhaft, den Katalysator bereits in der PCR-Chemie vorzuhalten. Für den letzteren Fall müssen PCR-kompatible Katalysatoren ausgewählt werden, d.h. Stoffe, die die PCR nicht negativ beeinflussen. Z.B. eignet sich hierzu Hydrochinon. Hierdurch wird die chemolumineszierende Substanz erzeugt, so dass deren chemische Energie auf die Fluorophore übertragen wird. Sofern die Fluorophore in Folge der erfolgten Amplifikationsreaktion räumlich von ihren Quenchern getrennt sind, ist die Fluoreszenz als Nachweis für die erfolgte Amplifikationsreaktion detektierbar. Insgesamt ist daher die Lichtemission der Fluoreszenz proportional zur Menge der Quencher-freien Fluorophore, die wiederherum proportional zur erfolgten Amplifikationsreaktion ist. Somit ist die Fluoreszenz-Lichtemission proportional zur Menge an gebildeten Produkten, so dass anhand der Fluoreszenz eine Auswertung der Amplifikationsreaktion möglich ist.

Die Erfindung umfasst weiterhin einen Kit zur Durchführung der beschriebenen Amplifikationsreaktion in einer mikrofluidischen Vorrichtung. Dieser Kit umfasst vorzugsweise zwei Reaktionsgemische, wobei das erste Reaktionsgemisch Komponenten für die Durchführung der Amplifikations-Reaktion und das zweite Reaktionsgemisch Komponenten für die Durchführung der Nachweisreaktion enthält.

Das erste Reaktionsgemisch enthält vor allem die Ausgangssubstanzen, die für einen Fluoreszenz-Readout der Reaktion geeignet sind und die insbesondere mit einem oder mehreren Fluorophoren und Quenchern markiert sind, beispielsweise entsprechend markierte Primer oder Sonden. Darüber hinaus kann das erste Reaktionsgemisch übliche Substanzen für die Durchführung einer Amplifikationsreaktion enthalten, beispielsweise für eine übliche PCR-Reaktion oder eine isothermalen Amplifikationsreaktion, z. B. Puffer, Nukleotide, Primer und/oder DNA-Polymerase. Diese üblichen Substanzen für die Durchführung der Amplifikationsreaktion können fakultativ enthalten sein oder, je nach Anwendungsfall, vom Anwender bereitgestellt und zugegeben werden. Darüber hinaus kann das erste Reaktionsgemisch auch einen PCR-kompatiblen Katalysator für die spätere Oxidationsreaktion (Nachweisreaktion) enthalten. Für die Durchführung der Reaktion kann es auch vorgesehen sein, dass der Anwender den PCR-kompatiblen Katalysator einem Standard-PCR-Kit für die spätere Oxidationsreaktion zugibt. Darüber hinaus kann das erste Reaktionsgemisch bereits wenigstens eine chemolumineszierende Substanz enthalten, mit Hilfe derer die Nachweisreaktion gemäß dem vorgeschlagenen Verfahren durchgeführt wird. Als chemolumineszierende Substanz wird insbesondere Luminol, verwendet. Das Oxidationsmittel, wie z.B. Wasserstoffperoxid oder Carbamidperoxid, wird separat bzw. im zweiten Reaktionsgemisch des Kits bereitgestellt, da es die Amplifikationsreaktion stören würde.

Das zweite Reaktionsgemisch des Kits stellt Komponenten bereit, welche vorzugsweise auch die Substanzen enthalten, die für die Reaktion sorgen, mit der die Anregung der chemolumineszierenden Substanzen erfolgt. Hierbei handelt es sich insbesondere um die Substanzen für eine Oxidationsreaktion, beispielsweise Wasserstoffperoxid oder Carbamidperoxid als Oxidationsmittel und gegebenenfalls ein oder mehrere Katalysatoren für die Oxidationsreaktion und/oder geeignete Puffersubstanzen, beispielsweise zum Einstellen von geeigneten alkalischen Bedingungen. Wie bereits erwähnt kann der Katalysator, sofern PCR-kompatibel, auch bereits Bestandteil des zuvor beschriebenen ersten Reaktionsgemischs des Kits sein. Unter Verwendung der Reaktionsgemische des Kits kann in der oben beschriebenen Weise die Amplifikationsreaktion durchgeführt und ausgewertet werden, wobei mit Hilfe der chemolumineszierenden Substanz chemische Energie auf die Fluorophore übertragen wird, die daraufhin Licht (Fluoreszenz) emittieren können, sofern kein Quencher benachbart ist. Bezüglich weiterer Merkmale des Kits zur Durchführung der Amplifikationsreaktion wird auf die obige Beschreibung verwiesen.

Schließlich umfasst die Offenbarung, ohne dass dies zur Erfindung gehört eine mikrofluidische Vorrichtung zur Durchführung von Amplifikationsreaktionen. Diese Vorrichtung ist dadurch gekennzeichnet, dass sie für die Durchführung des vorgeschlagenen Verfahrens eingerichtet ist. Bei dieser Vorrichtung kann es sich insbesondere um einen Lab-on-a-chip handeln, der einen Silizium-Chip mit einer Vielzahl von Mikroarrayzellen enthält, wie sie zur Durchführung von miniaturisierten Reaktionen, beispielsweise molekulardiagnostischen Reaktionen in der medizinischen Diagnostik, bereits bekannt sind. Diese mikrofluidische Vorrichtung ist zur Durchführung einer Amplifikationsreaktion mit einem Fluoreszenz-*Readout* eingerichtet, wobei im Rahmen der Reaktion Ausgangssubstanzen verwendet werden, die mit einem oder mehreren Fluorophoren und einem Quencher markiert sind. Dabei erfolgt die Anregung der Fluoreszenz nicht, wie bei herkömmlichen Verfahren, durch optische Anregung, sondern durch eine Energie-übertragende Reaktion aus chemolumineszierenden Molekülen, so dass chemische Energie auf die Fluorophore übertragen wird, die zur Emission der Fluoreszenzstrahlung führt, sofern kein Quencher in der Nachbarschaft des Fluorophors vorliegt. Die mikrofluidische Vorrichtung, die zur Durchführung dieser kombinierten Reaktion eingerichtet ist, zeichnet sich insbesondere dadurch aus, dass beispielsweise ein Katalysator und/oder ein Oxidationsmittel, die für die Reaktion zur Anregung der chemolumineszierenden Substanzen erforderlich sind, vorgelegt sind. Weiterhin können entsprechende Puffer vorgelegt sein. In der Regel werden die chemolumineszierenden Substanzen erst nach Abschluss der Amplifikationsreaktion im Sinne einer Endpunkt-Reaktion zugegeben, beispielsweise durch Überschichten mit einer entsprechenden Reaktionsflüssigkeit, die die entsprechenden Substanzen enthält. Bezüglich weiterer Merkmale dieser mikrofluidischen Vorrichtung wird auf die obige Beschreibung verwiesen.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Zeichnungen zeigen:
- Fig. 1: Spektren der Absorption und Emission des Fluorophors Yakima-Yellow, und
- Fig. 2: Emissionsspektrum von Luminol.

### Beschreibung von Ausführungsbeispielen

Das vorgeschlagene Verfahren stellt ein neues Verfahren zur Durchführung einer Amplifikationsreaktion in einer mikrofluidischen Vorrichtung bereit, bei dem die an sich bekannte Nachweismethode für die Amplifikationsreaktion auf der Basis von Fluoreszenzfarbstoffen mit einer strahlungslosen Anregung der Fluorophore durch Energieübertrag aus angeregten Molekülen, verknüpft wird. Bei der an sich bekannten Fluoreszenzmethode, die im Zusammenhang mit Amplifikationsreaktionen eingesetzt wird, werden mit einem Fluorophor markierte Ausgangssubstanzen, beispielsweise Fluorophor-markierte Primerketten oder TaqMan-Sonden während der Amplifikationsreaktion eingebaut oder anhybridisiert oder hydrolysiert. Diese Primer oder Sonden enthalten ein oder mehrere Fluorophore und einen Quencher, welche unmittelbar benachbart sind. Solange diese Konstellation aus Fluorophor und unmittelbar benachbartem Quencher besteht, kann keine Fluoreszenzemission erfolgen. Insbesondere kann keine Fluoreszenzemission aus solchen jeweils Fluorophor und Quencher tragenden Primerketten oder Sonden erfolgen, solange sich diese frei in der Lösung befinden. Erst durch den Einbau von derartigen Primerketten oder Sonden in ein Amplifikationsprodukt kommt es entweder zur Aufspaltung und räumlichen Trennung von Fluorophor und Quencher oder zur Abspaltung des Quenchers von dem das Fluorophor tragenden Molekül. Solche Fluorophore können nun nach Anregung Fluoreszenzstrahlung emittieren und stellen ein Maß für die Menge an gebildeten Amplifikationsprodukten dar. Mit diesem Verfahren kann durch die Energie-Übertragung auf die Fluorophore auf der Basis einer chemischen Reaktion auf aufwendige und teure Optiken verzichtet werden, die herkömmlicherweise für eine optische Anregung im Rahmen der Fluoreszenz-Nachweisreaktion erforderlich waren.

Das vorgeschlagene Verfahren kann insbesondere für eine Endpunkt-PCR-Reaktion oder eine isothermale Endpunkt-Amplifikationsreaktion im Singleplex oder Multiplex in einem Silizium-Mikroarray oder in einer anderen mikrofluidischen Vorrichtung durchgeführt werden, wobei während der Amplifikationsreaktion der beschriebene Einbau von Molekülpaaren aus Quenchern und Fluorophoren und/oder die Hydrolyse von derartigen Ausgangssubstanzen während der Bildung der Amplifikationsprodukte erfolgt. Nach Ablauf der Amplifikationsreaktion, also sobald die Amplifikationsreaktion in den Arrayzellen im Wesentlichen an ihrem Endpunkt angekommen ist, kann die mikrofluidische Vorrichtung mit einer Reaktionsflüssigkeit überschichtet werden, die die chemolumineszierenden Substanzen enthält. Diese Substanzen können nach ihrer Anregung über eine längere Zeitspanne chemische Energie tragen (unabhängig von deren Lumineszenz), so dass diese Energie auf die Fluorophore strahlungslos übertragen werden kann.

Die der Anregung der chemolumineszierenden Substanzen zugrunde liegende Reaktion, insbesondere die Oxidationsreaktion, kann durch einen Katalysator beschleunigt werden. Hierfür eignet sich insbesondere rotes Blutlaugensalz (Kaliumhexacyanoferrat-III) oder Hydrochinone oder andere Oxidationskatalysatoren. Dieser Katalysator kann dem Reaktionsgemisch aus Wasserstoffperoxid, Luminol und geeigneten Puffern beigemischt werden. Manche Katalysatoren können auch direkt in den Arrayzellen der mikrofluidischen Vorrichtung vorgelagert werden, sofern sie die eigentliche Amplifikationsreaktion nicht stören, was z.B. für viele Hydrochinone der Fall ist. Andere Möglichkeiten für geeignete Katalysatoren sind beispielsweise Manganperoxid (Braunstein), das ebenfalls in den Arrayzellen insbesondere in Form einer Suspension vorgelagert sein kann. Manganperoxid ist nicht wasserlöslich und stört daher nicht die Amplifikationsreaktion. Bei Kontakt mit Wasserstoffperoxid katalysiert Manganperoxid den Zerfall der Wasserstoffperoxidmoleküle und die Oxidation der chemolumineszierenden Substanzen, beispielsweise von Luminol, zur langlebigen angeregten Form. Manganperoxid ist darüber hinaus eine sehr kostengünstige und ungefährliche Verbindung, die daher besonders als Feststoff-Katalysator für den Zerfall von Wasserstoffperoxid und die Initiierung der nachfolgenden Oxidation des Luminols geeignet ist.

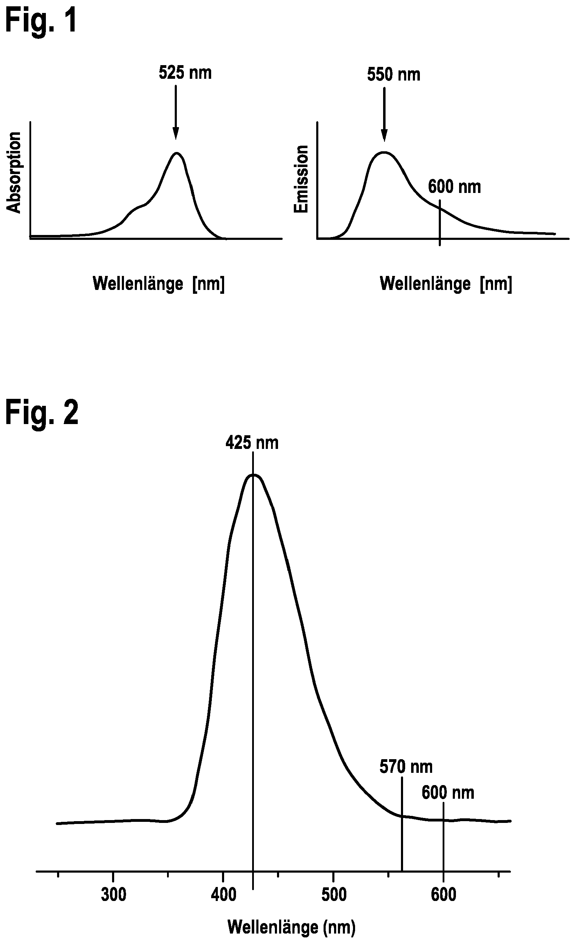

Als Fluorophore eignen sich im Prinzip alle Fluoreszenzfarbstoffe, die mit einer größeren Wellenlänge als die verwendete chemolumineszierende Substanz emittieren. Beispielsweise eignen sich als Fluorophore, die in Kombination mit Luminol als chemolumineszierender Substanz eingesetzt werden können, Rhodamin-B, andere Rhodamine, Yakima-Yellow oder Cy-5. **Fig. 1** illustriert Absorptions- und Emissionsspektren von Yakima-Yellow. Dabei liegt das Absorptionsmaximum bei 525 nm und das Emissionsmaximum bei 550 nm und die Emission erstreckt sich deutlich langwelliger bis über 600 nm hinaus. Das Emissionsspektrum von Luminol ist in **Fig. 2** dargestellt: Das Emissionsmaximum liegt bei 425 nm und die Emission erstreckt sich zu deutlich größeren Wellenlängen. Somit kann die Anregungsenergie von Luminol zu einer Anregung von Yakima-Yellow führen. Für die Auswertung der Fluoreszenzemission von Yakima-Yellow ist es vorteilhaft, dass beide Emissionen, also die störende Lumineszenzemission von Luminol und die Fluoreszenzemission von Yakima-Yellow durch optische Filter leicht voneinander getrennt werden können, so dass die Lumineszenz-Emission von Luminol nicht die für die Auswertung der Nachweisreaktion relevante Fluoreszenzemission stört bzw. überlagert.

Übliche Sonden mit dem Fluorophor Yakima-Yellow und einem Quencher basieren im Prinzip auf einer Brückenstruktur, die eine Kopplung des Quenchers mit dem Fluorophor realisiert. Bei einem *Anneal* bzw. einer Anlagerung einer derart markierten Sonde an einen DNA-Strang öffnet sich die Brücke, wodurch der Quencher räumlich von dem Fluorophor Yakima-Yellow getrennt wird.

Die Art des *Readouts* im Sinne einer Endpunkt-PCR oder einer isothermalen Endpunkt-Amplifikationsreaktion ist technisch ohne großen Aufwand umzusetzen. Die einzelne Reaktion erlaubt dabei zwar keine quantitative Aussage, aber bei den möglichen hohen Multiplexgraden ist das Merkmal "quantitativ" im Allgemeinen nicht mehr erforderlich. Durch den besonderen Vorteil des vorgeschlagenen Verfahrens, wobei auf eine optische Anregung der Probenvolumina verzichtet wird, können beispielsweise auch großflächige Silizium-Mikroarrays mit sehr vielen Arrayzellen und unterschiedlichen Nachweisreaktionen parallel prozessiert und ausgelesen werden.

Bei der Durchführung des Verfahrens kann zunächst eine an sich bekannte Amplifikationsreaktion mit Primern und/oder Sonden, die für einen Fluoreszenz-*Readout* geeignet sind, durchgeführt werden. Nach dem Erreichen des Endpunktes der Amplifikationsreaktion kann das Reaktionsgemisch für die erfindungsgemäße Nachweisreaktion, das beispielsweise aus Wasser, Luminol, Wasserstoffperoxid/Urea (Carbamidperoxid) und rotem Blutlaugensalz (Kaliumhexacyanoferrat-III) als Katalysator für die Reaktion, die zur Anregung der chemolumineszierenden Substanzen führt, besteht, in der mikrofluidischen Vorrichtung dem PCR-Multiarray-Chip langsam zugeführt werden, um so den Reaktionsansatz der Amplifikationsreaktion langsam zu überschichten. Zur Unterstützung der Nachweisreaktion, insbesondere zur Destabilisierung des Wasserstoffperoxids und zur Erhöhung der Löslichkeit von Luminol kann beispielsweise auch eine geringe Menge an Natronlauge oder alkalischem Puffer (z.B. Natriumhydrogencarbonat) zugegeben werden, wobei auch eine Vorlagerung von geeigneten Puffern in der mikrofluidischen Vorrichtung vorgesehen sein kann.

Die Substanzen für die Reaktion, die zur Anregung der chemolumineszierenden Substanzen dient, können zum Start der Nachweisreaktion direkt zugegeben werden oder teilweise bereits in den Arrayzellen oder auch an anderer Stelle im Lab-on-Chip-System vorgelagert werden. Es ist ein besonderer Vorzug solcher Lab-on-Chip-Systeme, dass darin alle benötigten Reagenzien an geeigneter Stelle und in geeigneter Weise vorgelagert werden können. Insbesondere ein PCR-kompatibler Katalysator wie Hydrochinon eignet sich für eine Vorlagerung in den Arrayzellen des Siliziumchips, da Hydrochinon die Amplifikationsreaktion nicht stört. Eine andere besonders geeignete Möglichkeit ist die Vorlagerung von Manganperoxid (Braunstein) als wasserunlöslicher Feststoff-Katalysator in den Arrayzellen. Auch Manganperoxid als wasserunlösliche Substanz stört die Amplifikationsreaktion, die in wässriger Lösung von statten geht, im Allgemeinen nicht. Da Manganperoxid ein sehr effizienter Zerfallskatalysator für Wasserstoffperoxid und damit für die Oxidation des Luminols ist, eignet sich auch Manganperoxid in besonderer Weise als Ausgangspunkt für die chemische Anregung der chemolumineszierenden Substanz, insbesondere von Luminol. Das angeregte Luminol überträgt seine Energie auf das Fluorophor, beispielsweise Yakima-Yellow. Sofern das Fluorophor Yakima-Yellow von seinem Quencher räumlich getrennt ist, also insbesondere, wenn der Yakima-Yellow-Primer oder die Yakima-Yellow-Sonde an einen DNA-Strang gebunden ist und der Quencher dadurch abgetrennt ist, kann Yakima-Yellow fluoreszieren. Für das Auslesen der Fluoreszenz-Strahlung wird in bevorzugter Weise ein optischer Filter (z.B. 570 - 580 nm) gegebenenfalls kombiniert mit einem zusätzlichen Kantenfilter, der kurzwelligere Emission als 580 nm sperrt, verwendet, um die für die Auswertung störendende Lumineszenz-Strahlung von Lumiol zu unterdrücken.

Als Reaktionsansätze für die Durchführung der PCR in den Arrayzellen werden beispielsweise handelsübliche proprietäre PCR-Kits von Herstellern verwendet, die selbst nicht Gegenstand der vorliegenden Erfindung sind. Der ausgewählte PCR-Kit wird gegebenenfalls ergänzt durch geeignete PCR-kompatible Katalysatoren und zusätzliche Puffersubstanzen, um ihn an die Umgebung der Arrayzellen im Siliziumchip optimal anzupassen. Darüber hinaus soll der PCR-Kit vorzugsweise mit Yakima-Yellow oder einem geeigneten Rhodamin als Nachweisfluorophor im Sinne der vorliegenden Beschreibung arbeiten.

Ein beispielhafter Reaktionsansatz für die Überschichtung des PCR-Kits nach Abschluss der PCR in den Arrayzellen zur Auslösung der Nachweisreaktion kann folgende Komponenten und Konzentrationen aufweisen:
- Reinstwasser
- Wasserstoffperoxid 0,1 % - 10% (entsprechend 30 mMolar - 3 Molar), vorzugsweise 0,5%-5% (entsprechend 150 mMolar - 1,5 Molar)
- alternativ Carbamidperoxid (Wasserstoffperoxid - Urea) 30 mMolar - 3 Molar, vorzugsweise 150 mMolar - 1,5 Molar
- Natriumhydroxid 10 mMolar - 1 Molar, bevorzugt 50 - 500 mMolar, besonders bevorzugt 100-150 mMolar
- Alternativ Natriumhydrogencarbonat 50 mMolar - 1 Molar, bevorzugt 100-500 mMolar
- Luminol 10 mMolar - 150 mMolar, besonders bevorzugt 50 - 100 mMolar
- Katalysator (Hydrochinon, *Horse Radish Peroxidase* HRP, Manganperoxid, Kaliumhexacyanoferrat-III) 10 mMolar - 1 Molar, vorzugsweise 50 mMolar-500 mMolar, besonders bevorzugt 100 mMolar

## Patentansprüche

1. Verfahren zur Durchführung einer Amplifikationsreaktion in einer mikrofluidischen Vorrichtung, wobei die Reaktion unter Verwendung von mit Fluorophor und Quencher markierten Ausgangssubstanzen durchgeführt wird und wobei durch eine im Rahmen der Amplifikationsreaktion erfolgte Trennung von Fluorophor und Quencher ein Nachweis von Reaktionsprodukten erfolgt, **dadurch gekennzeichnet, dass** der Nachweis durch Zugabe von wenigstens einer chemolumineszierenden Substanz und Auswertung der erfolgenden Fluoreszenzemission der Fluorophore durchgeführt wird.

2. Verfahren nach Anspruch 1, **dadurch gekennzeichnet, dass** die chemolumineszierende Substanz 3-Aminophthalhydrazid und/oder 3-Nitrophthalhydrazid ist.

3. Verfahren nach einem der vorhergehenden Ansprüche, **dadurch gekennzeichnet, dass** der Nachweis in Gegenwart von Wasserstoffperoxid durchgeführt wird.

4. Verfahren nach Anspruch 3, **dadurch gekennzeichnet, dass** das Wasserstoffperoxid in Form von Carbamidperoxid eingesetzt wird.

5. Verfahren nach einem der vorhergehenden Ansprüche, **dadurch gekennzeichnet, dass** der Nachweis in Gegenwart von wenigstens einem Katalysator erfolgt.

6. Verfahren nach Anspruch 5, **dadurch gekennzeichnet, dass** der Katalysator Kaliumhexacyanoferrat-III und/oder Manganperoxid und/oder Hydrochinon und/oder Brenzcatechin und/oder Resorcin und/oder *Horse Radish Peroxidase* (HRP) ist.

7. Verfahren nach Anspruch 5 oder Anspruch 6, **dadurch gekennzeichnet, dass** der Katalysator in der mikrofluidischen Vorrichtung vorgelegt wird.

8. Verfahren nach einem der vorhergehenden Ansprüche, **dadurch gekennzeichnet, dass** die Zugabe der wenigstens einen chemolumineszierenden Substanz durch Überschichtung mit einer Reaktionsflüssigkeit erfolgt, in der die wenigstens eine chemolumineszierende Substanz enthalten ist.

9. Verfahren nach einem der vorhergehenden Ansprüche, **dadurch gekennzeichnet, dass** als Fluorophor Rhodamin-B, ein anderes Rhodamin und/oder Yakima-Yellow und/oder Cy-5 verwendet wird.

10. Verfahren nach einem der vorhergehenden Ansprüche, **dadurch gekennzeichnet, dass** die Auswertung unter Verwendung wenigstens eines optischen Filters erfolgt.

11. Verfahren nach einem der vorhergehenden Ansprüche, **dadurch gekennzeichnet, dass** die Amplifikationsreaktion eine Endpunkt-Reaktion ist.

12. Kit zur Durchführung einer Amplifikationsreaktion in einer mikrofluidischen Vorrichtung gemäß Anspruch 1, wobei mit Fluorophor und Quencher markierte Ausgangssubstanzen und wenigstens eine chemolumineszierende Substanz für eine Nachweisreaktion enthalten sind.

## Claims

1. Method for carrying out an amplification reaction in a microfluidic device, wherein the reaction is carried out using starting substances labelled with a fluorophore and a quencher and wherein a separation of the fluorophore and quencher that has taken place in the course of the amplification reaction allows reaction products to be detected, **characterized in that** the detection is carried out by adding at least one chemiluminescent substance and evaluating the fluorescence emission of the fluorophores that occurs.

2. Method according to Claim 1, **characterized in that** the chemiluminescent substance is 3-aminophthalhydrazide and/or 3-nitrophthalhydrazide.

3. Method according to either of the preceding claims, **characterized in that** the detection is carried out in the presence of hydrogen peroxide.

4. Method according to Claim 3, **characterized in that** the hydrogen peroxide is used in the form of carbamide peroxide.

5. Method according to any of the preceding claims, **characterized in that** the detection is performed in the presence of at least one catalyst.

6. Method according to Claim 5, **characterized in that** the catalyst is potassium hexacyanoferrate(III) and/or manganese peroxide and/or hydroquinone and/or catechol and/or resorcinol and/or horseradish peroxidase (HRP).

7. Method according to Claim 5 or Claim 6, **characterized in that** the catalyst is initially introduced into the microfluidic device.

8. Method according to any of the preceding claims, **characterized in that** the at least one chemiluminescent substance is added by overcoating with a reaction liquid in which the at least one chemiluminescent substance is contained.

9. Method according to any of the preceding claims, **characterized in that** the fluorophore used is Rhodamine B, another Rhodamine and/or Yakima Yellow and/or Cy5.

10. Method according to any of the preceding claims, **characterized in that** the evaluation is performed using at least one optical filter.

11. Method according to any of the preceding claims, **characterized in that** the amplification reaction is an endpoint reaction.

12. Kit for carrying out an amplification reaction in a microfluidic device according to Claim 1, wherein starting substances labelled with a fluorophore and a quencher and at least one chemiluminescent substance for a detection reaction are contained.

## Revendications

1. Procédé de mise en œuvre d'une réaction d'amplification dans un dispositif microfluidique, la réaction étant mise en œuvre à l'aide de substances de départ marquées par un fluorophore et un agent d'extinction et une séparation, ayant lieu dans le cadre de la réaction d'amplification, du fluorophore et de l'agent d'extinction permettant une détection de produits de réaction, **caractérisé en ce que** la détection est mise en œuvre par ajout d'au moins une substance chimioluminescente et par évaluation de l'émission de fluorescence du fluorophore qui a lieu.

2. Procédé selon la revendication 1, **caractérisé en ce que** la substance chimioluminescente est le 3-aminophthalhydrazide et/ou le 3-nitrophthalhydrazide.

3. Procédé selon l'une des revendications précédentes, **caractérisé en ce que** la détection est mise en œuvre en présence de peroxyde d'hydrogène.

4. Procédé selon la revendication 3, **caractérisé en ce que** le peroxyde d'hydrogène est utilisé sous forme de peroxyde de carbamide.

5. Procédé selon l'une des revendications précédentes, **caractérisé en ce que** la détection a lieu en présence d'au moins un catalyseur.

6. Procédé selon la revendication 5, **caractérisé en ce que** le catalyseur est l'hexacyanoferrate III de potassium et/ou le peroxyde de manganèse et/ou l'hydroquinone et/ou le pyrocatéchol et/ou le résorcinol et/ou la peroxydase de raifort (HRP).

7. Procédé selon la revendication 5 ou la revendication 6, **caractérisé en ce que** le catalyseur est disposé au préalable dans le dispositif microfluidique.

8. Procédé selon l'une des revendications précédentes, **caractérisé en ce que** l'ajout de ladite au moins une substance chimioluminescente a lieu par recouvrement par un liquide de réaction dans lequel ladite au moins une substance chimioluminescente est contenue.

9. Procédé selon l'une des revendications précédentes, **caractérisé en ce qu'**on utilise, comme fluorophore, la rhodamine-B, une autre rhodamine et/ou le Yakima-Yellow et/ou le Cy-5.

10. Procédé selon l'une des revendications précédentes, **caractérisé en ce que** l'évaluation a lieu à l'aide d'au moins un filtre optique.

11. Procédé selon l'une des revendications précédentes, **caractérisé en ce que** la réaction d'amplification est une réaction au point final.

12. Kit pour la mise en œuvre d'une réaction d'amplification dans un dispositif microfluidique selon la revendication 1, des substances de départ marquées par un fluorophore et un agent d'extinction et au moins une substance chimioluminescente pour une réaction de détection étant contenues.
